(19) Europäisches Patentamt
European Patent Office
Office européen des brevets

(11) EP 1 624 905 B1

(12) # EUROPÄISCHE PATENTSCHRIFT

(45) Veröffentlichungstag und Bekanntmachung des Hinweises auf die Patenterteilung:
**09.08.2006 Patentblatt 2006/32**

(21) Anmeldenummer: **04728734.7**

(22) Anmeldetag: **22.04.2004**

(51) Int Cl.:
***A61L 27/16*** (2006.01) ***C08K 5/15*** (2006.01)
***C08J 3/205*** (2006.01)

(86) Internationale Anmeldenummer:
**PCT/AT2004/000133**

(87) Internationale Veröffentlichungsnummer:
**WO 2004/101009 (25.11.2004 Gazette 2004/48)**

(54) **VERNETZTES, ULTRA-HOCHMOLEKULARES POLYETHYLEN (UHMW-PE)**
CROSSLINKED, ULTRAHIGH MOLECULAR WEIGHT POLYETHYLENE (UHMW-PE)
POLYETHYLENE RETICULE DE POIDS MOLECULAIRE TRES ELEVE (POLYETHYLENE UHMW)

(84) Benannte Vertragsstaaten:
**AT BE BG CH CY CZ DE DK EE ES FI FR GB GR HU IE IT LI LU MC NL PL PT RO SE SI SK TR**
Benannte Erstreckungsstaaten:
**HR LT LV**

(30) Priorität: **19.05.2003 AT 7692003**

(43) Veröffentlichungstag der Anmeldung:
**15.02.2006 Patentblatt 2006/07**

(73) Patentinhaber: **Lederer, Klaus**
**8775 Kalwang (AT)**

(72) Erfinder:
- **LEDERER, Klaus**
  **A-8775 Kalwang (AT)**
- **WOLF, Christian**
  **A-8010 Graz (AT)**

(74) Vertreter: **Schwarz, Albin et al**
**Wipplingerstrasse 32**
**1010 Wien (AT)**

(56) Entgegenhaltungen:
**EP-A- 0 995 449** **WO-A-00/49079**

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist. (Art. 99(1) Europäisches Patentübereinkommen).



Printed by Jouve, 75001 PARIS (FR)

**Beschreibung**

Die vorliegende Erfindung betrifft ein neuartiges vernetztes, ultra-hochmolekulares Polyethylen (UHMW-PE). Ferner betrifft die Erfindung Formkörper aus diesem UHMW-PE sowie Verfahren, durch welche dieses erhältlich ist.

Ca. 70% aller weltweit eingesetzten Hüft- und Knieendoprothesen besitzen Gleitflächen aus UHMW-PE. Obwohl sich diese nunmehr seit mehr als 30 Jahren erfolgreich im klinischen Einsatz befinden, ist ihre Lebensdauer meist auf 10 bis 15 Jahre begrenzt. Grund für diese Lebensdauerbegrenzung sind oxidative Schädigungsmechanismen des UHMW-PE im menschlichen Körper, in folge derer der PE-Abrieb dramatisch zunehmen kann und es zu Entzündungen in der Umgebung des Implantats kommt. In den meisten Fällen macht dies eine aufwendige Revisionsoperation erforderlich.

Umfangreiche Untersuchungen (C. Wolf et al., J.Mat.Sci.: Mat. in Med. 13 (2002), 185-189; C. Wolf et al., J.Mat.Sci.: Mat. in Med. 13 (2002), 701-705)) haben gezeigt, dass die Schädigung von UHMW-PE-Gleitflächen infolge Oxidation durch die Beigabe des natürlichen Antioxidans α-Tocopherol (Vitamin E) deutlich verzögert wird, wodurch eine Verlängerung der Lebensdauer derartiger Prothesen um den Faktor 2,5 erwartet werden kann.

In der EP 0 995 449 A wird ein solches UHMW-Polyethylen beschrieben, das durch Ablagerung von in einer Flüssigkeit gelöstem oder suspendiertem Vitamin E auf dem pulverförmigen Ausgangsmaterial dotiert wird.

Gemäß der WO 00/49079 A wird ein mit einem Antioxidans, wie α-Tocopherol, dotiertes UHMW-PE dadurch hergestellt, dass die Polyethylenteilchen mit dem Antioxidans und $CO_2$ unter superkritischen Fluid-Bedingungen unter erhöhter Temperatur und erhöhtem Druck gemischt werden, um ein superkritisches Gemisch zu bilden, und dieses danach entspannt wird, wodurch das $CO_2$ verdampft.

In beiden der oben beschriebenen Verfahren wird das dotierte UHMW-PE anschließend durch Extrudieren, Verpressen und dergleichen zu Stangen und Blöcken für die weitere Bearbeitung geformt.

In den letzten Jahren ist mit strahlenvernetztem UHMW-PE ein weiterer Werkstoff für Gleitflächen von Endoprothesen in Erscheinung getreten. Durch die Behandlung mit hochenergetischer Strahlung kann das Abriebverhalten von UHMW-PE deutlich verbessert werden. Anschließend an den Bestrahlungsvorgang wird ein spezieller Temperschritt nachgeschaltet, der zu einer weiteren Vernetzung sowie zu einer Absättigung der durch die Bestrahlung entstandenen freien Radikale führt. Da die freien Radikale als Startpunkt für die Oxidation angesehen werden, führt dies zu einer erhöhten Oxidationsbeständigkeit des Materials. Betrachtet man jedoch die chemische Struktur des vemetzten UHMW-PE, erkennt man, dass es mit dem bisher verwendeten Standard-UHMW-PE chemisch praktisch ident ist (das vernetzte UHMW-PE weist allerdings tertiäre C-Atome als Vernetzungsstellen auf, welche einem oxidativen Angriff sogar noch zugänglicher sind als sekundäre C-Atome der Hauptkette). Eine Beimengung von α-Tocopherol sollte demnach die Oxidationsbeständigkeit von vernetztem UHMW-PE deutlich erhöhen.

Im Unterschied zu herkömmlichem UHMW-PE ist es bei strahlenvernetztem PE nicht möglich, α-Tocopherol vor der eigentlichen Verarbeitung in das PE-Pulver einzumischen, da es während des Bestrahlungsvorgangs und auch während des Temperprozesses die Vernetzung verhindert und dabei selbst in einem hohen Maß abgebaut wird.

Die Erfindung stellt sich die Aufgabe, ein vernetztes, ultra-hochmolekulares Polyethylen bereitzustellen, dessen Oxidationsbeständigkeit erhöht ist, d.h. das stabilisiert wurde. Hierdurch soll insbesondere eine höhere Lebensdauer von Formkörpern aus diesem UHMW-PE, besonders solchen, die als Endoprothesen ausgebildet sind, erreicht werden. Weiters ist es Aufgabe der Erfindung, ein Verfahren bereit zu stellen, durch welches das erfindungsgemäße UHMW-PE erhältlich ist.

Die erste Aufgabe wird erfindungsgemäß durch ein vernetztes, ultra-hochmolekulares Polyethylen gelöst, welches α-Tocopherol als Stabilisator enthält.

Das erfindungsgemäße Verfahren, durch welches das α-Tocopherol enthaltende UHMW-PE erhältlich ist, ist dadurch gekennzeichnet ist, dass das α-Tocopherol in das vernetzte ultra-hochmolekulare Polyethylen eindiffundieren gelassen wird.

Aufgrund der Verhinderung der Vernetzung des Polyethylen und des Abbaus von α-Tocopherol kann dessen Einbringung in das UHMW-PE nur im Anschluss an die Fertigungsschritte am bestrahlten Halbzeug oder am fertigen Endprodukt erfolgen. Es hat sich gezeigt, dass das physikalische Transportphänomen der Diffusion zielführend eingesetzt werden kann, um α-Tocopherol in das vernetzte UHMW-PE einzubringen. Die Konzentration von α-Tocopherol lässt sich hierbei durch Variation der Prozessparameter (Temperatur, Diffusionszeit) beeinflussen.

In einer bevorzugten Ausführungsform des Verfahrens wird das Eindiffundieren unter Inertgasatmosphäre durchgeführt.

Das Eindiffundieren des α-Tocopherols wird bevorzugt bei einer Temperatur im Bereich von 100 bis 200°C durchgeführt.

Vorzugsweise wird das Polyethylen nach dem Eindiffundieren des α-Tocopherols unter Inertgasatmosphäre getempert. Dies führt zu einer gleichmäßigen Verteilung des α-Tocopherols im Polyethylen, das durch Sorption und Diffusion zuerst im Wesentlichen nur in den Rand- und Oberflächenbereichen des vernetzten UHMW-PE eingebracht wird.

Die Temperung des Polyethylen wird vorzugsweise bei einer Temperatur im Bereich von 160 bis 200°C durchgeführt.

Gemäß einer bevorzugten Ausführungsform der Erfindung wird das α-Tocopherol bei 130°C 60 Minuten lang in das Polyethylen eindiffundieren gelassen und wird dieses anschließend bei 200°C 24 Stunden getempert.

Das erfindungsgemäße vernetzte, ultra-hochmolekulare Polyethylen ist ferner dadurch erhältlich, dass das α-Tocopherol in Gegenwart von überkritischem $CO_2$ in das Polyethylen eindiffundieren gelassen wird.

$CO_2$ lässt sich relativ leicht in den überkritischen Zustand überführen und diffundiert aufgrund seiner geringen molekularen Abmessungen schnell in Polymere ein. Es ist zudem ungiftig, nicht brennbar, umweltschonend und preisgünstig. Vitamin E ist nach folgendem Zusammenhang (W. Chrastil, J.Phys.Chem. 86(15), 1982, 3016-3021) in überkritischem $CO_2$ löslich:

$$c = d^{8,231} \cdot e^{(-17353,5/T + 0,646)}$$

wobei c die Konzentration [g/l] von α-Tocopherol in $CO_2$, d [g/l] die Dichte von $CO_2$ und T die Temperatur in K darstellt.

Vorzugsweise wird das Eindiffundieren des α-Tocopherols in das Polyethylen bei einer Temperatur im Bereich von 100 bis 180°C durchgeführt.

Das Eindiffundieren des α-Tocopherols in das Polyethylen wird bevorzugt bei einem Druck im Bereich von 150 bis 300 bar durchgeführt.

Die beim Eindiffundieren des α-Tocopherols in das Polyethylen eingestellte Temperatur wird vorzugsweise während des Entspannungsvorgangs beibehalten, um Gefügeänderungen des UHMW-PE zu vermeiden.

Gemäß einem weiteren Aspekt betrifft die Erfindung Formkörper aus dem erfindungsgemäßen UHMW-PE, insbesondere solche, die als Endoprothese ausgebildet sind.

Die Erfindung wird nachfolgend anhand der Beispiele und der Figuren 1-9 näher erläutert, wobei Fig. 5 das Oxidationsprofil eines erfindungsgemäßen Formkörpers zeigt und die Figuren 1-4 und 6-9 jeweils ein Diagramm darstellen, in dem die Konzentration von α-Tocopherol über den Querschnitt eines erfindungsgemäßen Formkörpers aufgetragen ist.

Material und allgemeine Messmethodik

Bei dem in den Untersuchungen verwendeten bestrahlten UHMW-PE handelte es sich um Durasul der Fa. Centerpulse, Winterthur, Schweiz. Als Form für die Probekörper wurden Würfel mit einer Kantenlänge von 20 mm gewählt.

Zur Ermittlung der Verteilung des α-Tocopherols wurden die Würfel im Anschluss an die Versuche in der Mitte durchgeschnitten und von einer Schnittfläche mit Hilfe eines Mikrotomschnittgeräts eine Folie mit einer Dicke von ca. 200 μm abgehobelt. An dieser Folie wurde dann mit einem FTIR-Mikroskop in der Mitte von einem Rand zum anderen ein Linescan mit einem Messpunkteabstand von 300 μm durchgeführt. Die α-Tocopherol-Konzentration an den jeweiligen Messpunkten wurde durch das Verhältnis des vom α-Tocopherol verursachten Peaks bei 1230-1279 $cm^{-1}$ zu dem PE-Peak bei 2020 $cm^{-1}$ ermittelt. Diese Konzentration wurde über dem Abstand von den Seitenkanten der Folie in einem Diagramm aufgetragen und so das Konzentrationsprofil dargestellt. Die Einheit der Konzentrationsangaben ist Masse α-Tocopherol zu Masse UHMW-PE in Prozent. Zusätzlich wurden die Würfel vor und nach den Versuchen gewogen und die Gesamtkonzentration an α-Tocopherol bestimmt.

Einbringung von α-Tocopherol in vemetztes UHMW-PE durch Diffusion

Es wurde versucht, α-Tocopherol durch einfaches Eindiffundieren in die Durasul-Würfel einzubringen. Hierzu wurden die Würfel in einen Autoklaven eingebracht und dieser mit reinem α-Tocopherol befüllt. Anschließend wurde der Autoklav 10 Minuten lang mit Stickstoff gespült, um eine Inertgasatmosphäre zu erzeugen. Nach Ablauf der Spülphase wurde der Autoklav verschlossen und ein Druck von ca. 15 bar über die Stickstoffzufuhr eingestellt. Eine Variation des Drucks im Autoklavengefäß zeigte keinen wesentlichen Einfluss auf die Diffusionsgeschwindigkeit. In mehreren Versuchen wurden die wesentlichen Einflussparameter Temperatur sowie Diffusionsdauer variiert.

Ergebnisse:

Im ersten Teil dieser Versuchsreihe wurde die maximale Temperatur auf 100°C festgelegt. Der Grund hierfür ist, dass vemetztes UHMW-PE bis zu dieser Temperatur ausreichend formstabil ist, d.h. man kann so α-Tocopherol in ein fertiges Endprodukt einbringen, ohne dass weitere Nachbearbeitungsschritte notwendig sind. Es wurde daher nur die Versuchszeit, d.h. die Diffusionsdauer, variiert.

Die gewählten Versuchsparameter sind Tabelle 1 zu entnehmen, die dazugehörigen Konzentrationsprofile sind in Fig. 1 dargestellt.

Tabelle 1

| Versuch | Temperatur [°C] | Zeitdauer [h] | Gesamtmenge α-Tocopherol [% w/w] |
|---|---|---|---|
| DIFF04 | 100 | 24 | 1,27 |
| DIFF05 | 100 | 48 | 1,62 |
| DIFF06 | 100 | 96 | 1,82 |

Zwar wurden keine allzu großen Eindringtiefen in einem vernünftigen Zeitrahmen realisiert (siehe Fig. 1), es wurde jedoch die in erster Linie von der Oxidation betroffene Randschicht mit variierbarer Stabilisatorkonzentration versehen und so ein Schutz vor oxidativem Abbau geschaffen. Diese Methode ist daher vor allem für fertige Produkte geeignet.

In einer zweiten Versuchsreihe wurden die Versuche bei erhöhter Temperatur durchgeführt. Insbesondere bei Erreichen des Kristallitschmelzbereichs konnte eine deutliche Steigerung der Diffusionsgeschwindigkeit sowie der absorbierten Masse an α-Tocopherol beobachtet werden.

Kristalline Bereiche im PE stellen für das α-Tocopherol Diffusionsbarrieren dar; die Wanderung der Moleküle findet praktisch ausschließlich in der amorphen Phase statt. Durch Aufschmelzen der kristallinen Bereiche kommt es daher - zusätzlich zum beschleunigenden Effekt der erhöhten Temperatur - zu der beobachteten starken Steigerung der Diffusionsgeschwindigkeit.

Durch die Erhöhung der Temperatur kommt es ferner zu einer starken Erhöhung der Löslichkeit des α-Tocopherols im UHMW-PE. Bei 200°C konnten bis zu 40% w/w α-Tocopherol in das Polyethylen eingebracht werden. Allerdings liegen diese Werte weit über der Sättigungskonzentration bei Raumtemperatur, das Material war daher bei Zimmertemperatur stark mit α-Tocopherol "überladen". Dies bewirkte nach dem Abkühlen ein verstärktes Ausdiffundieren des Vitamin E, die Würfel "schwitzten" sehr stark.

Aus Fig. 1 und den oben beschriebenen Versuchen ist ersichtlich, dass die Temperatur die Rand- bzw. Sättigungskonzentration sowie die Eindringtiefe maßgeblich beeinflusst, wohingegen mit der Diffusionszeit in erster Linie die Eindringtiefe eingestellt werden kann.

Die Erhöhung der Temperatur beschleunigt den Diffusionsvorgang, wie oben beschrieben, zwar erheblich, doch steigt auch die Randkonzentration weit über den Bereich einer vernünftigen Stabilisatorkonzentration von ca. 0,2 bis max. 1 % an. Ohne eine Verdünnung des α-Tocopherols und somit der Einführung einer weiteren Komponente kann keine homogene Vitamin E-Verteilung in einem vernünftigen Zeitrahmen und Konzentrationsbereich mittels einfacher Diffusion bewerkstelligt werden. Hinsichtlich einer Verwendung als Implantatwerkstoff ist jedoch jede Einführung einer neuen Komponente zu vermeiden, da dies den erforderlichen Zulassungsprozess erheblich erschweren kann.

Zum Erreichen einer gleichmäßigen Verteilung wurde daher anschließend an die Diffusion ein Temperprozess durchgeführt. Hierzu wurden die Proben, wie oben beschrieben, zuerst im Autoklaven mit α-Tocopherol versetzt, dann aus diesem herausgenommen und anschließend in einem Kolben unter Stickstoffatmosphäre bei unterschiedlichen Temperaturen für unterschiedlich lange Zeitintervalle gelagert. Im Autoklaven wurde somit mittels Temperatur eine je nach Diffusionszeit unterschiedlich dicke Randschicht mit einer hohen α-Tocopherol-Konzentration erzeugt und diese dann im anschließenden Temperprozess über den Würfel verteilt. Tabelle 2 und die Figuren 2, 3 und 4 zeigen die Versuchsparameter bzw. die damit erhaltenen Konzentrationsprofile der getemperten Proben.

Tabelle 2

| | Diffusion | | Temperung | |
|---|---|---|---|---|
| Versuch | Temperatur [°C] | Diffusionsdauer [h] | Temperatur [°C] | Temperzeit [h] |
| DIFF09 | 130 | 2 | | |
| DIFF09_T160_22 | 130 | 2 | 160 | 22 |
| DIFF09_T160_44 | 130 | 2 | 160 | 44 |
| DIFF09_T200_22 | 130 | 2 | 200 | 22 |
| DIFF10 | 130 | 1 | | |
| DIFF10_T200_24 | 130 | 1 | 200 | 24 |

(fortgesetzt)

| | Diffusion | | Temperung | |
|---|---|---|---|---|
| Versuch | Temperatur [°C] | Diffusionsdauer [h] | Temperatur [°C] | Temperzeit [h] |
| DIFF11_T200_6 | 130 | 1,25 | 200 | 6 |

Zusätzlich wurden die CO-Zahlen ermittelt, um eine eventuelle Schädigung des Materials im Zuge des Temperprozesses festzustellen. Fig. 5 zeigt das Oxidationsprofil des PE-Würfels des Versuchs DIFF11_T200_6. Bei sorgfältiger Aufrechterhaltung der Inertatmosphäre konnte keine oxidative Schädigung des Materials festgestellt werden.

Aufgrund der erzielten Ergebnisse ist ersichtlich, dass durch eine Variation der Parameter Diffusionszeit, Temperatur während der Diffusion, Dauer und Temperatur des Temperprozesses das Konzentrationsprofil von α-Tocopherol in UHMW-PE in weiten Grenzen nach Belieben eingestellt werden kann.

Das Konzentrationsprofil DIFF10_T200_24 in Fig. 4 zeigt z.B. eine annähernd homogene Durchtränkung des UHMW-PE-Würfels mit einer Stabilisatorkonzentration von ca. 0,4 % w/w, was für viele Anwendungen als optimale Menge zu betrachten ist.

Herstellung eines homogen durchtränkten Würfels mit einer Konzentration von 0,4 % w/w α-Tocoyherol

Der Autoklav wird im kalten Zustand mit α-Tocopherol befüllt und der Würfel derart eingebracht, dass er vollkommen von α-Tocopherol umgeben ist. Anschließend wird der Autoklav verschlossen und 15 Minuten lang mit Stickstoff gespült. Nach Beendigung der Spülphase wird ein Druck von 15 bar aufgebracht und der Autoklav auf 130°C erwärmt. Mit Erreichen der 130°C wird die Temperatur für 60 Minuten konstant gehalten und dann auf Raumtemperatur abgekühlt.

Der Würfel wird aus dem Autoklaven genommen und in ein Glasgefäß gelegt, welches 15 Minuten lang mit Stickstoff gespült wird. Das Gefäß wird verschlossen und bei 200°C 24 Stunden lang gelagert. Nach Ablauf dieser Zeitspanne wird auf Raumtemperatur abgekühlt und der Würfel aus dem Gefäß entfernt.

Einbringung von α-Tocopherol in vernetztes UHMW-PE mittels überkritischem $CO_2$

Für die Versuche wurde ein Laborautoklav mit einem Fassungsvermögen von 300 ml verwendet, welcher sich zur Beheizung in einem Ofen befand. Die Temperatur wurde mit einem Fühler, der an der Autoklavenaußenwand angebracht war, ermittelt. An den Autoklaven angeschlossen waren eine $CO_2$-Hochdruckpumpe sowie ein mechanisches Auslassventil.

Die UHMW-PE-Würfel wurden zusammen mit der eingewogenen Menge an α-Tocopherol auf einem Gittergestell in den Autoklaven eingebracht. Anschließend wurde der Autoklav in den Ofen gestellt und langsam auf die gewünschte Temperatur aufgeheizt, wobei gleichzeitig der $CO_2$-Druck aufgebracht wurde. Nach Erreichen der gewünschten Temperatur wurde die Zeitnehmung gestartet. Nach Ablauf der gewünschten Versuchszeit wurde mit dem Entspannungsvorgang begonnen.

Das Entspannen ist mit einer derartigen Geschwindigkeit und Temperatur durchzuführen, dass es weder zu einem "Zerreißen" des UHMW-PE-Formkörpers noch zu Änderungen des Kristallgefiiges kommt.

Beim Entspannungsprozess wurde folgendermaßen vorgegangen: Nach Ablauf der vorgegebenen Versuchszeit wurde der Autoklav von der $CO_2$-Pumpe entkoppelt. Dies diente dazu, ein weiteres Nachfließen von kaltem Kohlendioxid zu verhindern. Da nur ein mechanisches Ventil zur Verfügung stand, war es nicht möglich, die erforderliche langsame Druckverminderung kontinuierlich durchzuführen, daher wurde der Druck diskontinuierlich in Stufen abgesenkt. In einem Zeitrahmen von 24 Stunden wurde der Druck etwa jede Stunde in 30 Sekunden um 20 bar erniedrigt (mit einer längeren Unterbrechung über Nacht). Die Temperatur wurde dabei immer auf der Versuchstemperatur belassen, einerseits aufgrund der dadurch erhöhten Diffusionsgeschwindigkeit, andererseits um Gefügeänderungen durch ein Kristallinisieren des Materials bei einer hohen $CO_2$-Konzentration zu vermeiden. Die Temperatur wurde erst im Anschluss an den Entspannungsvorgang langsam (innerhalb von ca. 3 Stunden) auf Raumtemperatur gesenkt.

Mit geregelten Auslassventilen lässt sich der Entspannungsvorgang in zeitlicher Hinsicht optimieren, wobei eine gewisse Mindestdauer in jedem Fall notwendig ist.

Ergebnisse:

Ähnlich wie bei den Versuchen mittels herkömmlicher Diffusion bestimmen die Versuchstemperatur sowie die Versuchsdauer maßgeblich die Diffusionsgeschwindigkeit und die Eindringtiefe von α-Tocopherol in vernetztem UHMW-PE. Bei Überschreiten des Kristallitschmelzbereichs kommt es, wie bereits beschrieben, zu einer deutlichen Erhöhung der Geschwindigkeit.

Fig. 6 veranschaulicht den Einfluss der Temperatur auf die Einbringung des α-Tocopherol in das vernetzte UMW-PE. In Tabelle 3 sind die entsprechenden Versuchsparameter aufgelistet.

Tabelle 3

| Versuch | Temperatur [°C] | Versuchsdauer [h] | Druck [bar] |
|---|---|---|---|
| W5 | 100 | 4 | 300 |
| W9 | 150 | 4,25 | 300 |
| W13 | 170 | 4 | 300 |

Durch Veränderung der Versuchsdauer kommt es in erster Linie zu unterschiedlichen Eindringtiefen, wie aus Fig. 7 und Tabelle 4 ersichtlich.

Tabelle 4

| Versuch | Temperatur [°C] | Versuchsdauer [h] | Druck [bar] |
|---|---|---|---|
| W8 | 150 | 1 | 300 |
| W9 | 150 | 4,25 | 300 |
| W10 | 150 | 2 | 300 |

Unterschiedlich ist der Einfluss des Drucks, der bei der Einbringung des α-Tocopherols mit $CO_2$ eine entscheidende Rolle spielt. Der Druck beeinflusst die Dichte von $CO_2$ und somit nach der oben angegebenen Formel die Löslichkeit des α-Tocopherols im $CO_2$ sowie die Diffusionsgeschwindigkeit. Wie aus Fig. 8 deutlich ersichtlich, senkt eine Verminderung des Drucks die eindiffundierte Menge an Vitamin E beträchtlich. Der Druck für alle weiteren Versuche wurde deshalb auf 300 bar belassen. Tabelle 5 zeigt die Versuchsparameter zu Fig. 8.

Tabelle 5

| Versuch | Temperatur [°C] | Versuchsdauer [h] | Druck [bar] |
|---|---|---|---|
| W13 | 170 | 4 | 300 |
| W14 | 170 | 3 | 150 |

Der größte Unterschied zu der Einbringung mittels herkömmlicher Diffusion besteht darin, dass durch die α-Tocopherol-Vorlage im $CO_2$ die maximale Konzentration im Werkstoff eingestellt werden kann; das $CO_2$ agiert sozusagen als Verdünnungsmittel. Fig. 9 (die entsprechenden Versuchsparameter sind in Tabelle 6 angeführt) zeigt die Ergebnisse von Versuchen mit unterschiedlicher α-Tocopherol-Vorlage. Ein Absenken der α-Tocopherol-Konzentration im $CO_2$ führt zu einer Senkung der durchschnittlichen α-Tocopherol-Konzentration im UHMW-PE.

Tabelle 6

| Versuch | Temperatur [°C] | Versuchsdauer [h] | Druck [bar] | α-Tocopherol-Konzentration [g/l] |
|---|---|---|---|---|
| W15 | 171 | 12 | 300 | 0,97 |
| W19 | 168 | 13,5 | 300 | 0,45 |
| W24 | 172 | 14 | 300 | 0,424 |
| W25 | 172 | 7 | 300 | 0,224 |

Aus den erzielten Ergebnissen ist ersichtlich, dass α-Tocopherol mittels überkritischem $CO_2$ in vernetztes UHMW-PE als Stabilisator eingebracht werden kann. Durch eine Variation der vier Haupteinflussgrößen Versuchsdauer, Druck, Temperatur und α-Tocopherol-Konzentration im $CO_2$ lässt sich das Konzentrationsprofil von α-Tocopherol in UHMW-PE in weiten Grenzen nach Belieben einstellen.

Da das Maximum des Konzentrationsverlaufs nicht über die Temperatur sondern in erster Linie über die α-Tocopherol-Vorlage im $CO_2$ eingestellt wird, kann man im Sinn von kurzen Prozesszeiten hohe Temperaturen und $CO_2$-Drücke anwenden. Zur homogenen Durchtränkung eines UHMW-PE-Würfels mit 2 cm Kantenlänge sind bei 170°C und 300 bar $CO_2$-Druck dennoch 12 Stunden notwendig.

Besonderes Augenmerk ist auch auf den Entspannungsprozess zu richten, da zu schnelles Entspannen zur Zerstörung des Materials führen kann.

Herstellung eines homogen durchtränkten Würfels mit einer Konzentration von 0,4 % w/w α-Tocopherol

Der Würfel wird zusammen mit dem α-Tocopherol in den kalten Autoklaven eingebracht. Die Menge an Vitamin E wird derart eingewogen, dass die Konzentration im $CO_2$ 0,75 g/l entspricht. Danach werden die Temperatur und der Druck langsam (ca. innerhalb einer Stunde) auf 170°C bzw. 300 bar erhöht. Nach 12 Stunden bei 170°C und 300 bar wird mit dem Entspannungsvorgang begonnen. Hierzu wird bei konstant 170°C der Druck innerhalb von 24 Stunden abgebaut. Anschließend wird die Temperatur innerhalb von 3 Stunden auf Raumtemperatur gesenkt.

## Patentansprüche

1. Verfahren zur Stabilisierung von vernetztem, ultra-hochmolekularem Polyethylen mit α-Tocopherol, **dadurch gekennzeichnet, dass** das α-Tocopherol in das Polyethylen eindiffundieren gelassen wird.

2. Verfahren nach Anspruch 1, **dadurch gekennzeichnet, dass** das Eindiffundieren unter Inertgasatmosphäre durchgeführt wird.

3. Verfahren nach Anspruch 1 oder 2, **dadurch gekennzeichnet, dass** das Eindiffundieren des α-Tocopherols bei einer Temperatur im Bereich von 100 bis 200°C durchgeführt wird.

4. Verfahren nach einem der Ansprüche 1 bis 3, **dadurch gekennzeichnet, dass** das Polyethylen nach dem Eindiffundieren des α-Tocopherols unter Inertgasatmosphäre getempert wird.

5. Verfahren nach Anspruch 4, **dadurch gekennzeichnet, dass** die Temperung des Polyethylen bei einer Temperatur im Bereich von 160 bis 200°C durchgeführt wird.

6. Verfahren nach Anspruch 4 oder 5, **dadurch gekennzeichnet, dass** das α-Tocopherol bei 130°C 60 Minuten lang in das Polyethylen eindiffundieren gelassen wird und dieses anschließend bei 200°C 24 Stunden getempert wird.

7. Verfahren nach Anspruch 1, **dadurch gekennzeichnet, dass** das α-Tocopherol in Gegenwart von überkritischem $CO_2$ in das Polyethylen eindiffundieren gelassen wird.

8. Verfahren nach Anspruch 7, **dadurch gekennzeichnet, dass** das Eindiffundieren des α-Tocopherols in das Polyethylen bei einer Temperatur im Bereich von 100 bis 180°C durchgeführt wird.

9. Verfahren nach Anspruch 7 oder 8, **dadurch gekennzeichnet, dass** das Eindiffundieren des α-Tocopherols in das Polyethylen bei einem Druck im Bereich von 150 bis 300 bar durchgeführt wird.

10. Verfahren nach einem der Ansprüche 7 bis 9, **dadurch gekennzeichnet, dass** die beim Eindiffundieren des α-Tocopherols in das Polyethylen eingestellte Temperatur während eines Entspannungsvorgangs beibehalten wird.

11. Vernetztes, ultra-hochmolekulares Polyethylen, **dadurch gekennzeichnet, dass** es α-Tocopherol als Stabilisator enthält.

12. Formkörper aus vernetztem, ultra-hochmolekularem Polyethylen nach Anspruch 11.

13. Formkörper nach Anspruch 12, ausgebildet als Endoprothese.

## Claims

1. A process for the stabilization of crosslinked ultra-high molecular weight polyethylene with α-tocopherol, **characterized in that** the α-tocopherol is allowed to diffuse into the polyethylene.

2. A process according to claim 1, **characterized in that** the inward diffusion is carried out under an inert gas atmosphere.

3. A process according to claim 1 or 2, **characterized in that** the inward diffusion of α-tocopherol is carried out at a temperature ranging from 100 to 200°C.

4. A process according to any of claims 1 to 3, **characterized in that** the polyethylene is annealed under an inert gas atmosphere following the inward diffusion of α-tocopherol.

5. A process according to claim 4, **characterized in that** the annealing of the polyethylene is carried out at a temperature ranging from 160 to 200°C.

6. A process according to claim 4 or 5, **characterized in that** the α-tocopherol is allowed to diffuse into the polyethylene at 130°C for 60 minutes, which polyethylene is subsequently annealed at 200°C for 24 hours.

7. A process according to claim 1, **characterized in that** the α-tocopherol is allowed to diffuse into the polyethylene in the presence of supercritical $CO_2$.

8. A process according to claim 7, **characterized in that** the diffusion of α-tocopherol into the polyethylene is carried out at a temperature ranging from 100 to 180°C.

9. A process according to claim 7 or 8, **characterized in that** the diffusion of α-tocopherol into the polyethylene is carried out at a pressure ranging from 150 to 300 bar.

10. A process according to any of claims 7 to 9, **characterized in that** the temperature adjusted during the diffusion of α-tocopherol into the polyethylene is maintained during an expansion process.

11. A crosslinked ultra-high molecular weight polyethylene, **characterized in that** it contains α-tocopherol as a stabilizer.

12. A moulded body made of crosslinked ultra-high molecular weight polyethylene according to claim 11.

13. A moulded body according to claim 12, designed as an endoprosthesis.

**Revendications**

1. Procédé de stabilisation de polyéthylène réticulé de poids moléculaire très élevé avec de l'α-tocophérol, **caractérisé en ce que** l'on laisse l'α-tocophérol se diffuser dans le polyéthylène.

2. Procédé selon la revendication 1, **caractérisé en ce que** la diffusion s'effectue sous atmosphère de gaz inerte.

3. Procédé selon la revendication 1 ou 2, **caractérisé en ce que** la diffusion de l'α-tocophérol s'effectue à une température allant de 100 à 200°C.

4. Procédé selon l'une des revendications 1 à 3, **caractérisé en ce que** le polyéthylène est étuvé sous atmosphère de gaz inerte après diffusion de l'α-tocophérol.

5. Procédé selon la revendication 4, **caractérisé en ce que** l'étuvage du polyéthylène est réalisé à une température allant de 160 à 200°C.

6. Procédé selon la revendication 4 ou 5, **caractérisé en ce que** l'on laisse l'α-tocophérol se diffuser dans le polyéthylène pendant 60 minutes à 130°C et que celui-ci est ensuite étuvé à 200°C pendant 24 heures.

7. Procédé selon la revendication 1, **caractérisé en ce que** l'on laisse l'α-tocophérol se diffuser dans le polyéthylène en présence de $CO_2$ supercritique.

8. Procédé selon la revendication 7, **caractérisé en ce que** la diffusion de l'α-tocophérol dans le polyéthylène s'effectue à une température allant de 100 à 180°C.

9. Procédé selon la revendication 7 ou 8, **caractérisé en ce que** la diffusion de l'α-tocophérol dans le polyéthylène s'effectue à une pression allant de 150 à 300 bars.

**10.** Procédé selon l'une des revendications 7 à 9, **caractérisé en ce que** la température ajustée lors de la diffusion de l'α-tocophérol dans le polyéthylène est conservée pendant une opération de détente.

**11.** Polyéthylène réticulé de poids moléculaire élevé, **caractérisé en ce qu'**il comprend de l'α-tocophérol comme stabilisateur.

**12.** Corps moulé en polyéthylène réticulé de poids moléculaire élevé selon la revendication 11.

**13.** Corps moulé selon la revendication 12, prenant la forme d'une endoprothèse.

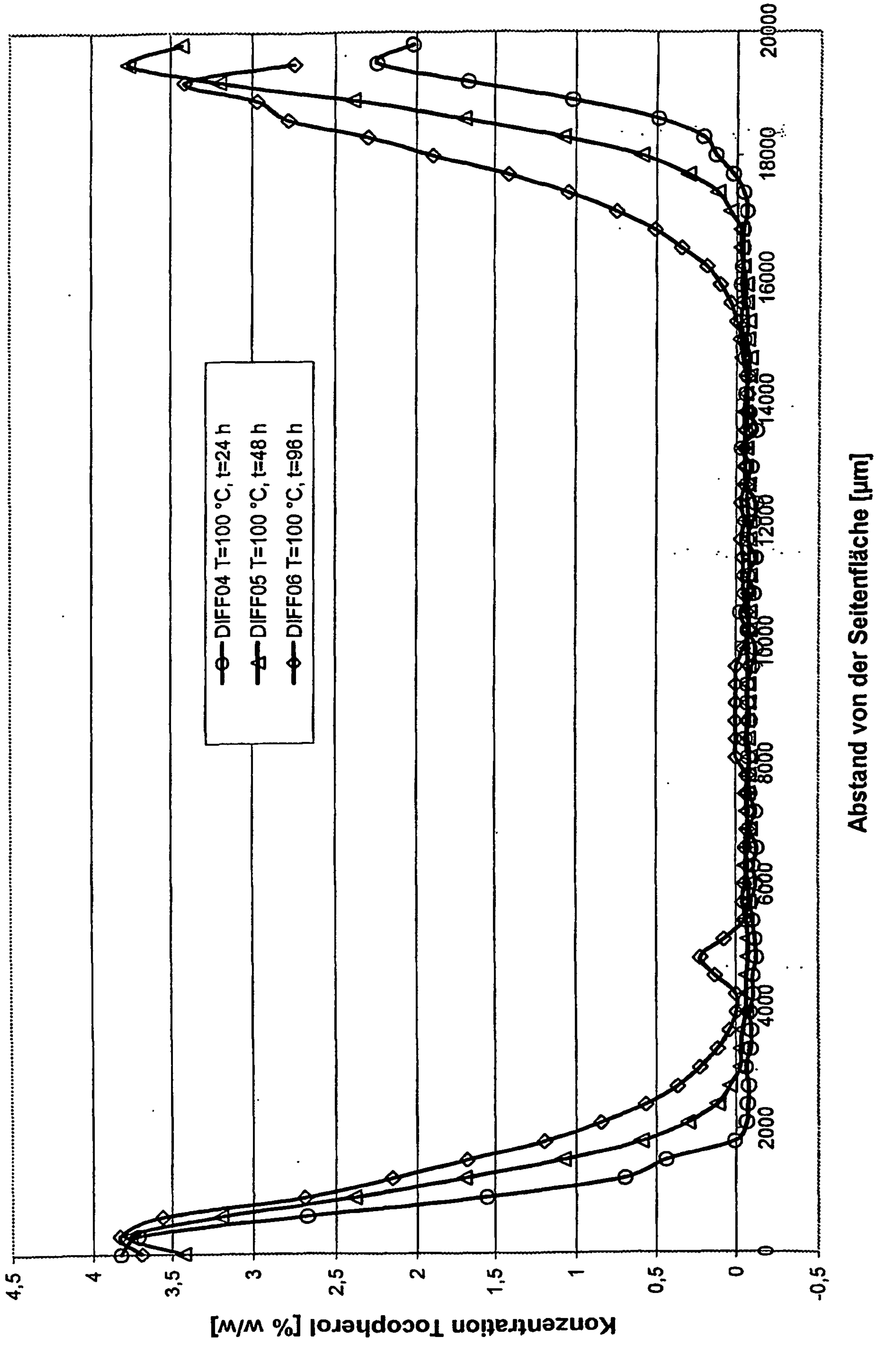
DIFF04 T=100 °C, t=24 h
DIFF05 T=100 °C, t=48 h
DIFF06 T=100 °C, t=96 h
Konzentration Tocopherol [% w/w]
4,5
4
3,5
3
2,5
2
1,5
1
0,5
0
-0,5
Abstand von der Seitenfläche [µm]
0
2000
4000
6000
8000
10000
12000
14000
16000
18000
20000

Fig. 1

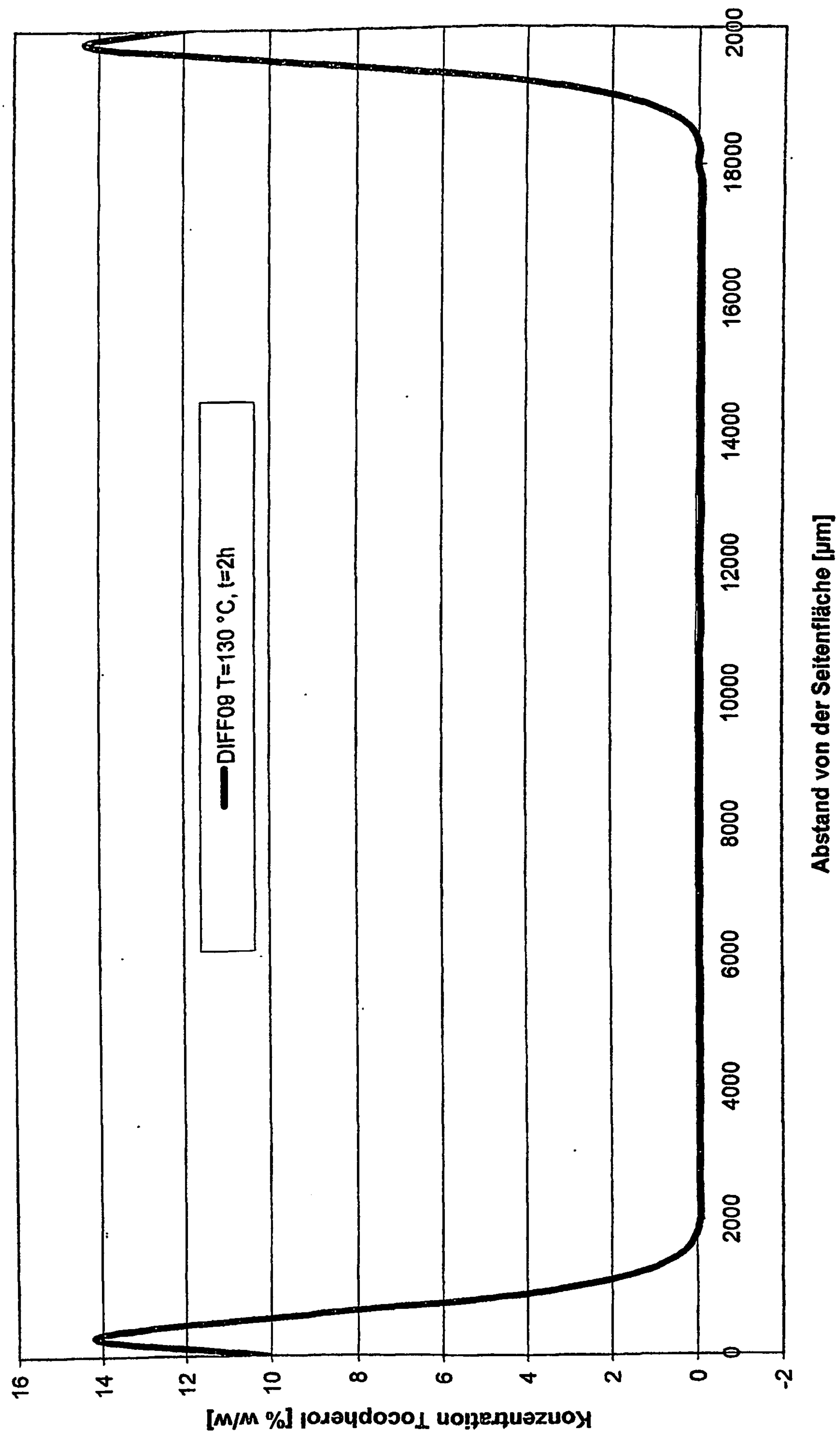
DIFF09 T=130 °C, t=2h
Konzentration Tocopherol [% w/w]
Abstand von der Seitenfläche [µm]
16
14
12
10
8
6
4
2
0
-2
0
2000
4000
6000
8000
10000
12000
14000
16000
18000
20000

Fig. 2

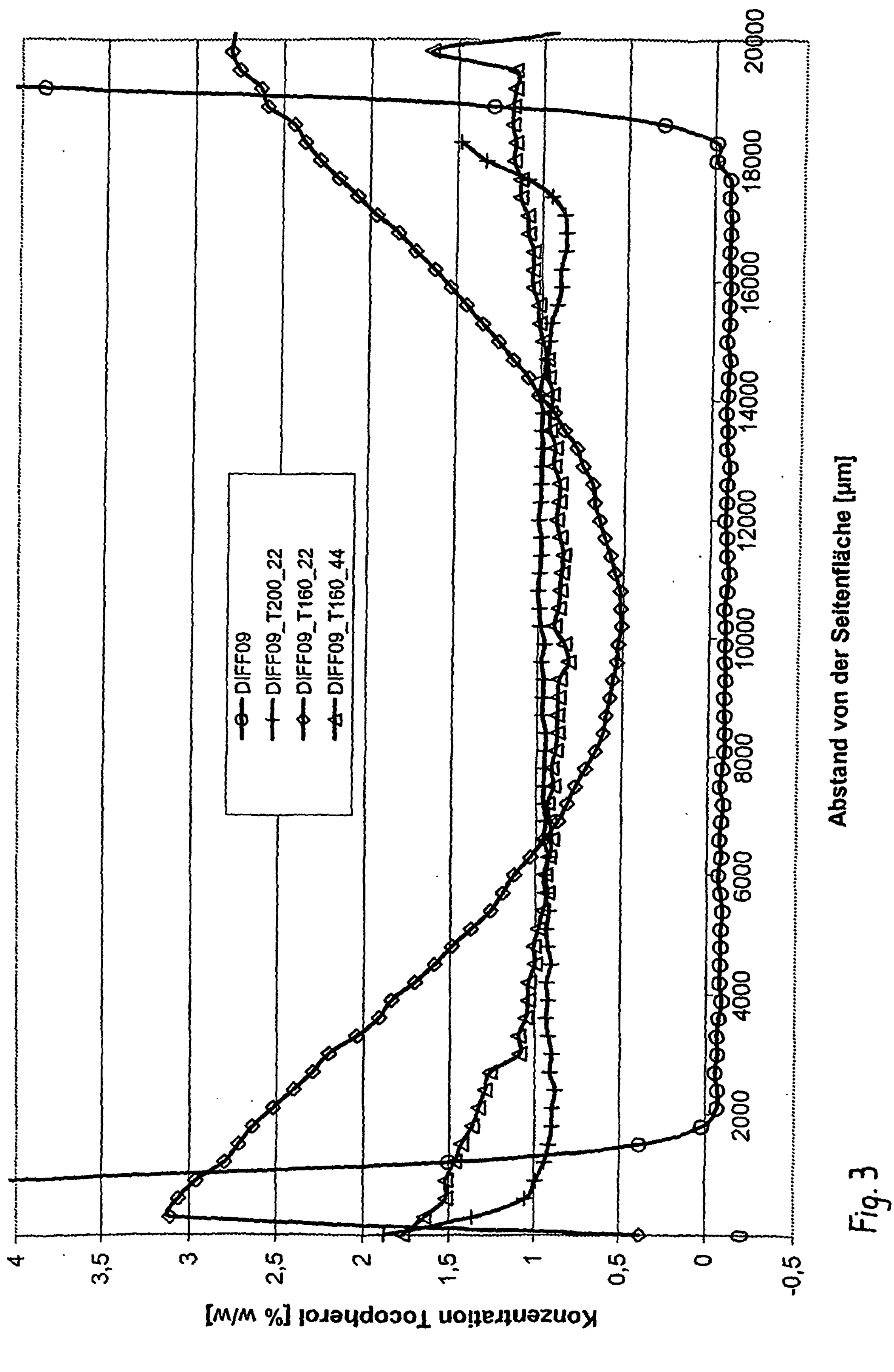
DIFF09
DIFF09_T200_22
DIFF09_T160_22
DIFF09_T160_44
Konzentration Tocopherol [% w/w]
Abstand von der Seitenfläche [µm]
4
3,5
3
2,5
2
1,5
1
0,5
0
-0,5
0
2000
4000
6000
8000
10000
12000
14000
16000
18000
20000

Fig. 3

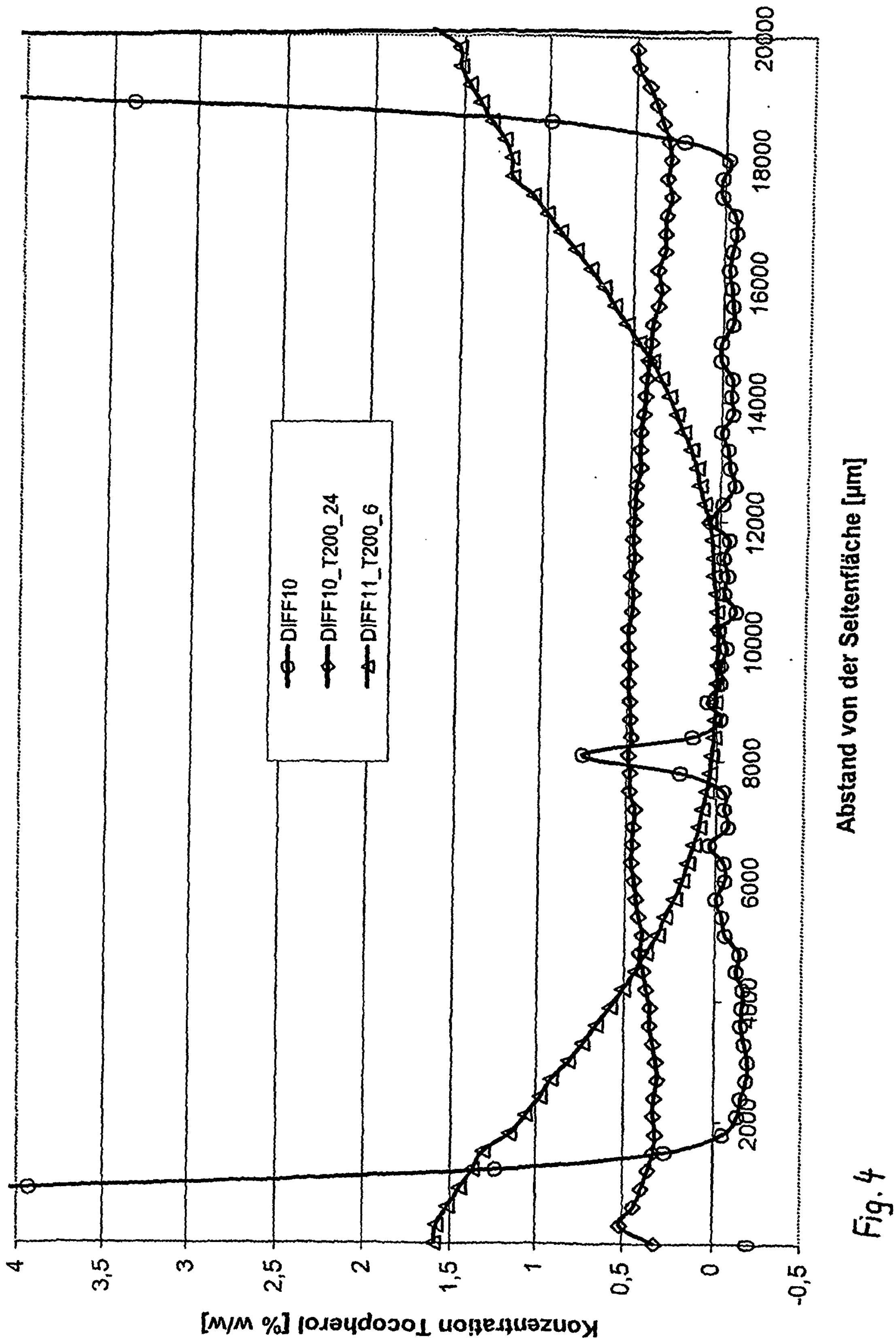

DIFF10
DIFF10_T200_24
DIFF11_T200_6
Konzentration Tocopherol [% w/w]
4
3,5
3
2,5
2
1,5
1
0,5
0
-0,5
Abstand von der Seitenfläche [µm]
0
2000
4000
6000
8000
10000
12000
14000
16000
18000
20000


Fig. 4

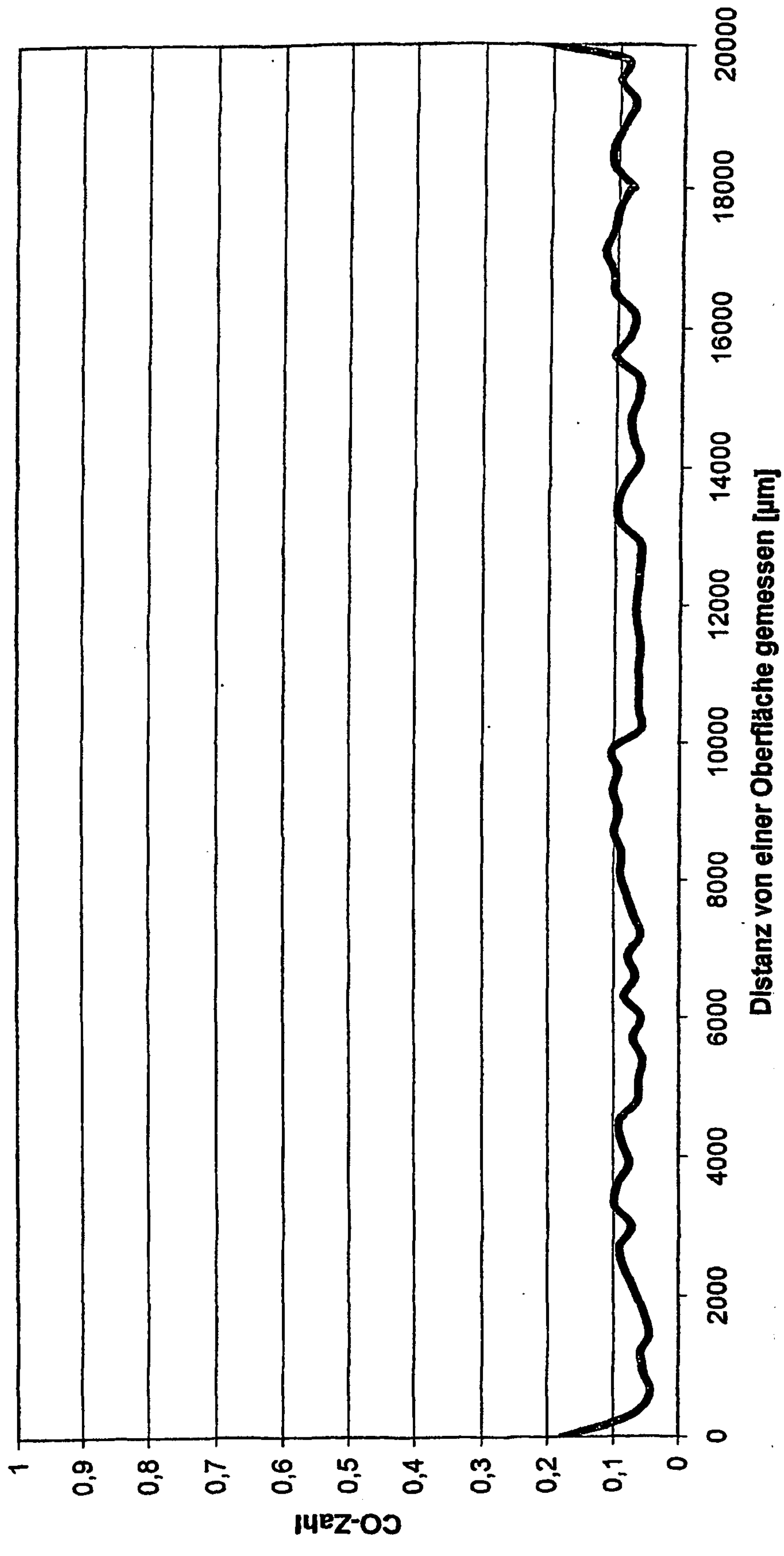
CO-Zahl
0
0,1
0,2
0,3
0,4
0,5
0,6
0,7
0,8
0,9
1
0
2000
4000
6000
8000
10000
12000
14000
16000
18000
20000
Distanz von einer Oberfläche gemessen [µm]

Fig. 5

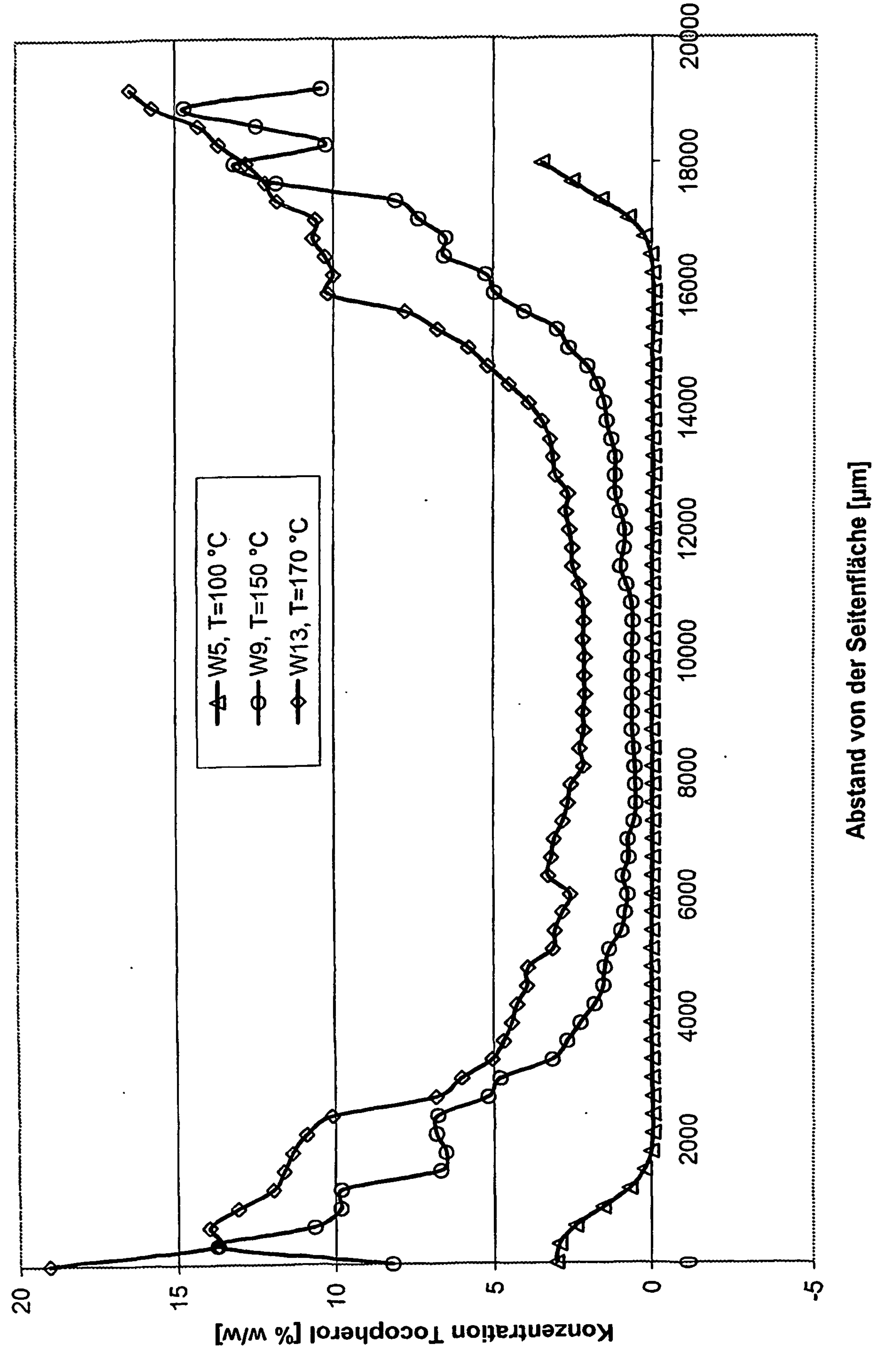

W5, T=100 °C
W9, T=150 °C
W13, T=170 °C
Konzentration Tocopherol [% w/w]
Abstand von der Seitenfläche [µm]
20
15
10
5
0
-5
0
2000
4000
6000
8000
10000
12000
14000
16000
18000
20000


Fig. 6

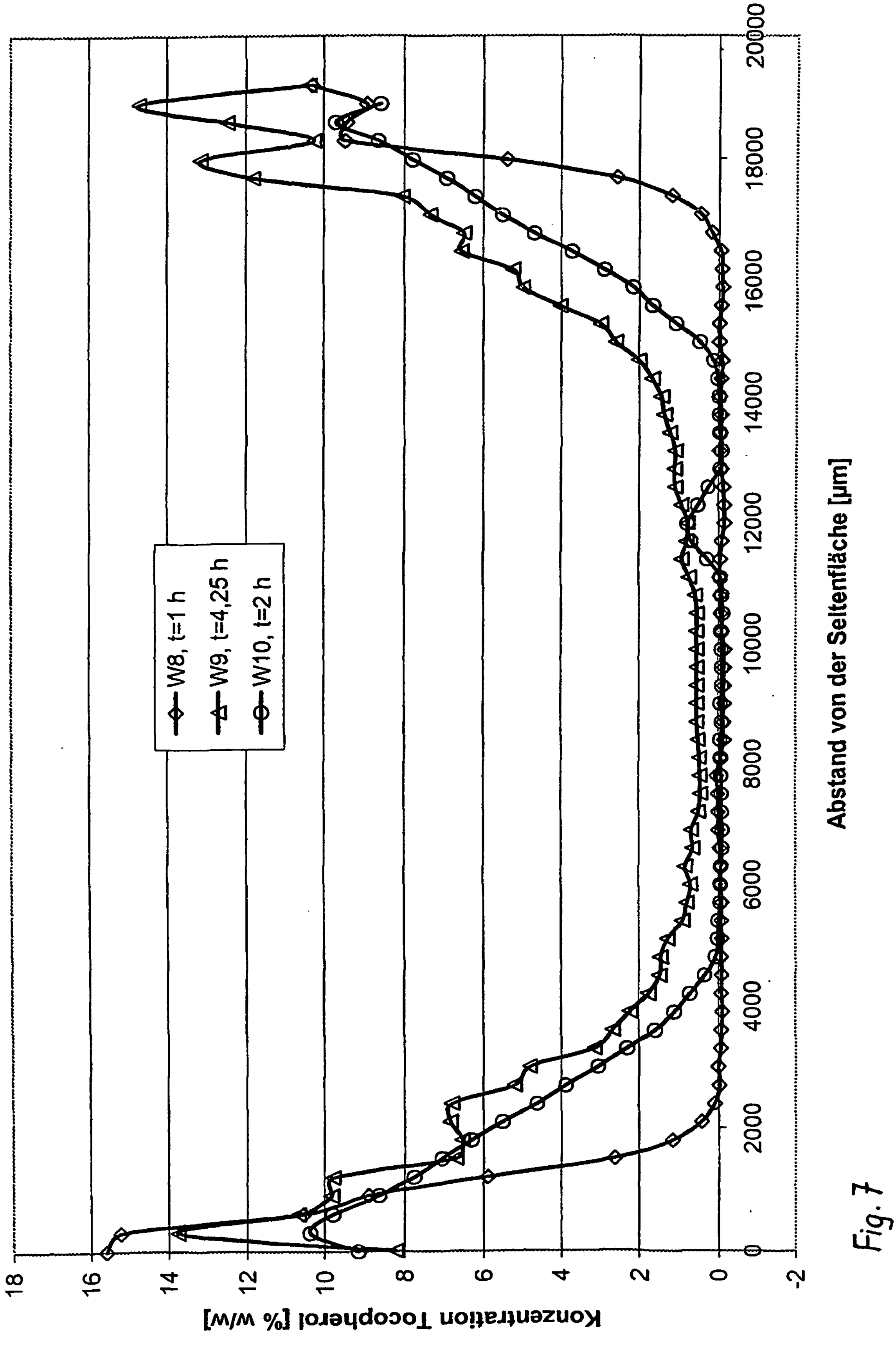
W8, t=1 h
W9, t=4,25 h
W10, t=2 h
Konzentration Tocopherol [% w/w]
Abstand von der Seitenfläche [µm]
18
16
14
12
10
8
6
4
2
0
-2
0
2000
4000
6000
8000
10000
12000
14000
16000
18000
20000

Fig. 7

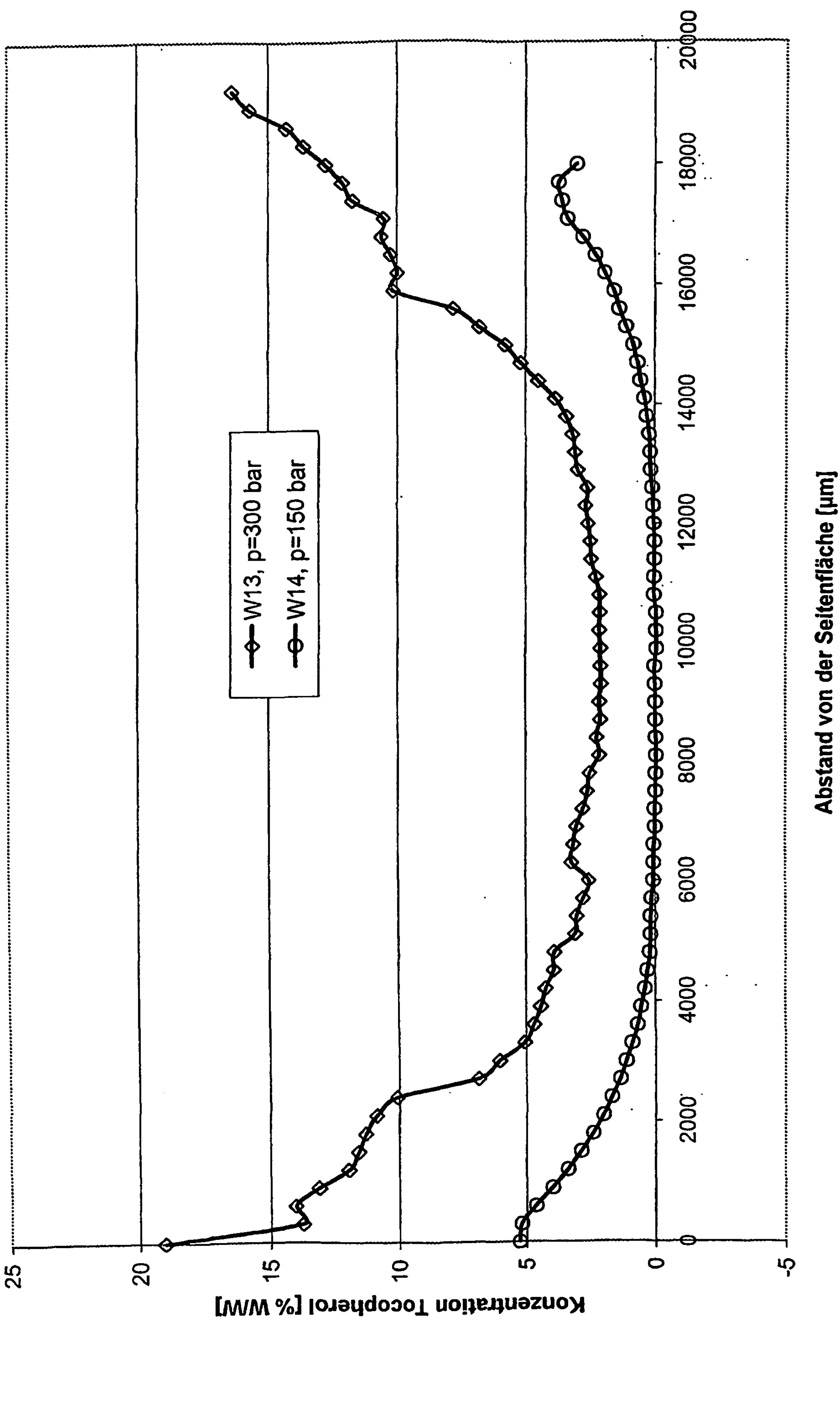
W13, p=300 bar
W14, p=150 bar
Konzentration Tocopherol [% W/W]
25
20
15
10
5
0
-5
Abstand von der Seitenfläche [µm]
0
2000
4000
6000
8000
10000
12000
14000
16000
18000
20000

Fig. 8

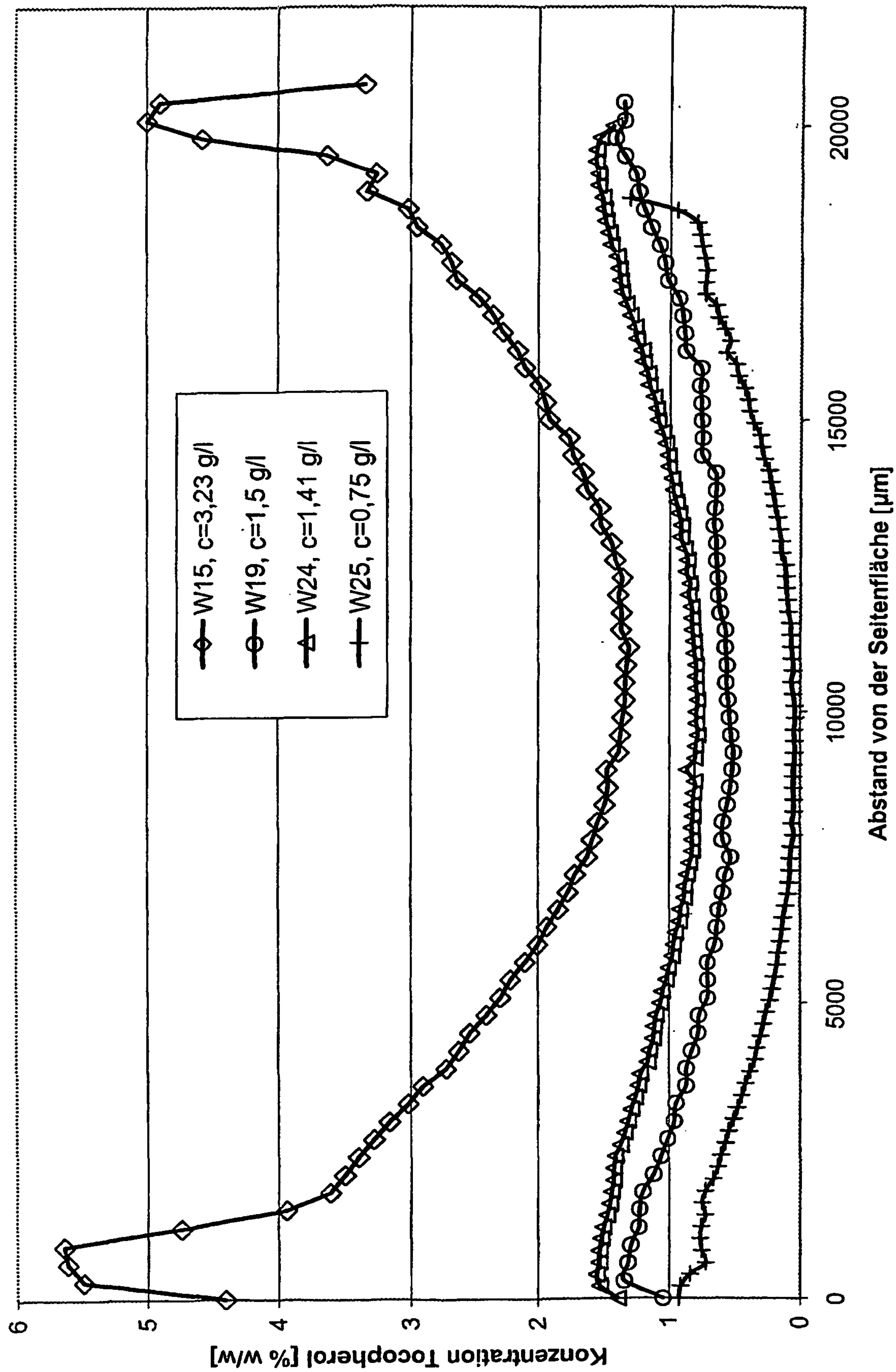
W15, c=3,23 g/l
W19, c=1,5 g/l
W24, c=1,41 g/l
W25, c=0,75 g/l
Konzentration Tocopherol [% w/w]
0
1
2
3
4
5
6
Abstand von der Seitenfläche [µm]
0
5000
10000
15000
20000

Fig. 9